# FASCICULE DE BREVET EUROPEEN

(11) **EP 1 419 733 B1**
(45) Date de publication et mention de la délivrance du brevet: **23.01.2008**
(21) Numéro de dépôt: 03292814.5
(22) Date de dépôt: 13.11.2003
(51) Int. Cl.: A61B 5/0452

(54) **Dispositif d'analyse d'un signal, notamment d'un signal physiologique tel qu'un signal ECG**
Vorrichtung zur Analyse eines Signales, insbesondere eines physiologischen Signals wie z.B. ein EKG-Signal
Device for analysis of a signal, in particular a physiological signal such as an ECG signal

(30) Priorité: 14.11.2002 FR 0214212
(43) Date de publication de la demande: 19.05.2004
(73) Titulaire: ELA MEDICAL, 92541 Montrouge (FR)
(72) Inventeur: Quenet, Brigitte, 75005 Paris (FR); Dubois, Rémi, 75006 Paris (FR); Faisandier, Yves, 75116 Paris (FR)
(74) Mandataire: Dupuis-Latour, Dominique

(56) Documents cités:
- EP-A- 1 219 237
- WO-A-02/07594
- US-A- 5 623 935
- US-A- 5 778 881
- US-B1- 6 309 342
- F.B. Vialatte: "Aide au diagnostic d'anomalies cardiaques"; Mémoire pour le DEA de Sciences Cognitives, Université Pierre et Marie Curie, Paris VI, 21 juin 2002, page 1-57

## Description

L'invention concerne, de façon générale, l'analyse de données physiologiques, par exemple les données recueillies par un appareil autonome ambulatoire ou un dispositif médical actif implanté.

Les dispositifs médicaux actifs sont définis par la directive 93/42/CE du 14 juin 1993 du Conseil des communautés européennes, les dispositifs médicaux implantables actifs étant quant à eux définis par la directive du Conseil 90/385/CE du 20 juin 1990.

L'invention sera particulièrement décrite dans le cas de l'analyse de signaux d'activité cardiaque, en particulier de signaux recueillis par un enregistreur dit "Holter", c'est-à-dire un appareil permettant d'effectuer en continu et sur une longue période l'enregistrement de signaux recueillis au moyen d'électrodes implantées (électrogrammes) ou d'électrodes externes (électrocardiogrammes), signaux qui seront désignés simplement par la suite "signaux ECG".

Cette application, à laquelle l'invention s'applique de façon particulièrement avantageuse, n'est cependant pas limitative et l'invention peut être appliquée aussi bien à l'analyse d'autres paramètres physiologiques tels qu'électroencéphalogramme, rythme respiratoire, tension artérielle, etc., ou non physiologiques tels que signaux radar, ultrasonores, etc.

Si l'on prend le cas de l'analyse d'un enregistrement Holter, celui-ci exige un travail de dépouillement assez complexe. En effet, l'enregistrement en continu des signaux ECG d'un patient sur 24 ou 48 heures représente environ 100 000 complexes PQRST, qu'il est nécessaire d'analyser ainsi que leur variabilité de manière à rechercher tout évènement pathologique tel que trouble du rythme, anoxie cardiaque, anomalie d'entraînement d'un stimulateur cardiaque, etc. Cette analyse, qui est effectuée de façon automatique par des algorithmes mis en oeuvre dans un ordinateur ou un appareil ambulatoire, fournit des résultats intermédiaires, synthétiques, à partir desquels le médecin pourra, ensuite, établir un diagnostic.

Ces algorithmes, du fait notamment du volume important de données qui doivent être traitées, nécessitent des moyens de calcul relativement importants, de sorte que leur optimisation, en termes d'efficacité par rapport aux ressources de calcul requises, est un facteur important dans le domaine de l'analyse des signaux physiologiques, notamment des signaux Holter.

Une autre difficulté réside dans le taux d'erreur à l'analyse des signaux, qui peut avoir des conséquences graves, avec notamment des risques de faux diagnostic. En effet, dans le cas particulier d'un ECG enregistré en ambulatoire, le signal n'est pas régulier et présente de nombreuses artefacts. Plus précisément, le signal ECG est constitué le plus souvent du signal d'origine cardiaque, presque périodique (complexe PQRST), accompagné de signaux générés par les muscles, par les perturbations mécaniques de l'interface électrode-peau, ou de perturbations électriques ou encore électromagnétiques reçues par les câbles reliant les électrodes à l'enregistreur.

Les algorithmes classiques savent assez mal éliminer la totalité de ces parasites et, même s'ils permettent d'atteindre typiquement un taux d'erreur de 0,1 %, ceci représente environ 100 erreurs sur un enregistrement de 24 heures (100 000 complexes PQRST), ce qui constitue un niveau d'erreur encore trop élevé, car ces erreurs peuvent être concomitantes à des complexes présentant des singularités importantes du point de vue du diagnostic.

Enfin, toujours pour un signal ECG, il est important de pouvoir observer la variabilité du complexe QRS, qui peut être très significative pour le diagnostic ; l'algorithme d'analyse utilisé doit donc pouvoir révéler et discriminer un certain nombre de microvariations.

L'analyse automatique d'un signal ECG comporte trois étapes distinctes, qui sont : 1°) le filtrage préalable du signal, de manière à éliminer un certain nombre de parasites dans le domaine fréquentiel et délivrer un signal de meilleure qualité ; 2°) la décomposition et l'identification des ondes caractéristiques du signal ; et 3°) une synthèse de l'évolution temporelle des paramètres décrivant ces différentes ondes caractéristiques.

Ces résultats permettent au médecin d'établir son diagnostic, et il est évident qu'ils doivent être à la fois fiables et pertinents pour faciliter ce diagnostic.

La fiabilité repose en partie sur la robustesse et la bonne adaptation de la décomposition et de l'identification réalisées à la deuxième étape.

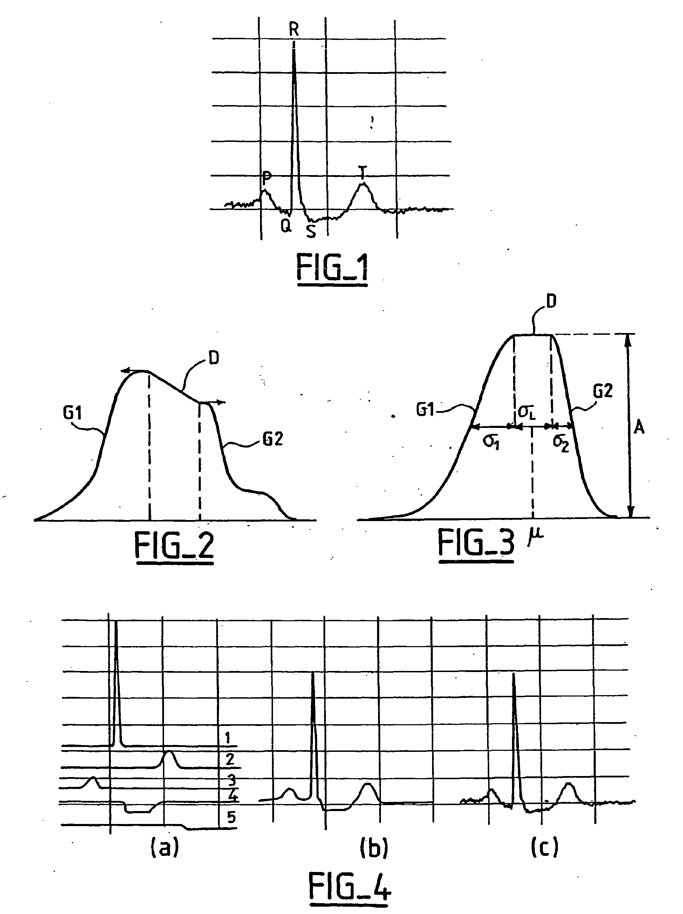

Plus précisément, le signal ECG se présente comme illustré figure 1, qui est un tracé représentant l'évolution au cours du temps de l'activité électrique du coeur, avec une succession d'ondes, d'amplitude positive ou négative, de part et d'autre de la ligne dite "isoélectrique" caractéristique du repos cardiaque.

Lors d'un battement cardiaque normal (celui illustré figure 1), ces ondes positives ou négatives sont identifiées comme résultant de processus physiologiques bien définis, ce qui permet d'attribuer à chacune des ondes un label standardisé, typiquement P,Q,R,S ou T. Physiologiquement, l'onde P est engendrée par la dépolarisation de l'oreillette, les ondes QRS, par la dépolarisation du ventricule et l'onde T, par la repolarisation de ce ventricule.

C'est à partir de la forme et de la position temporelle de ces diverses ondes, ainsi que de leur variabilité, que le médecin pourra reconnaître une pathologie.

Plusieurs procédés de décomposition et d'identification des ondes caractéristiques du signal ont été proposés.

Ainsi, l'analyse fréquentielle permet de décrire le signal dans l'espace de Fourier. Cependant, une telle décomposition n'est pas totalement adaptée à l'analyse d'un signal ECG car ce signal n'est pas rigoureusement périodique. Son contenu spectral, riche, varie dans le temps, et en outre les fonctions sinusoïdales de la décomposition ne permettent pas d'obtenir la phase du signal, nécessaire à l'identification des ondes ; il manque en effet à cette décomposition fréquentielle l'information temporelle.

Pour réaliser une décomposition temps-fréquence, on a proposé d'utiliser la transformée en ondelettes orthogonales, l'identification des ondes se faisant alors sur le contenu temps-fréquence des ondelettes qui modélisent le signal. Mais le manque de résolution de ce type de méthode devient rapidement une limite à l'analyse fine.

Cette difficulté peut être palliée par une décomposition en ondelettes non orthogonales (ou en fonctions radiales de base, ou en gaussiennes, etc.) : le signal ECG est décomposé en une somme de gaussiennes qui sont de taille soit fixe, soit adaptable. Cette méthode a souvent été utilisée mais souffre d'un handicap caractéristique tenant au fait que les ondes à modéliser (c'est-à-dire les ondes P,Q,R,S et T) ne sont pas vraiment des gaussiennes, de sorte que leur modélisation nécessite l'emploi d'un très grand nombre de paramètres pour être de qualité suffisante, nécessitant alors une puissance de calcul considérable pour pouvoir être mis en oeuvre dans un temps raisonnable. De plus, si le résultat est correct pour l'onde QRS, l'onde P et l'onde T, qui sont difficilement assimilable à des gaussiennes, sont assez mal modélisées, sauf à employer un grand nombre de paramètres pour obtenir une qualité suffisante conduisant ainsi à une complexification excessive de l'algorithme d'analyse.

Finalement, pour ces diverses raisons, on utilise le plus souvent une décomposition linéaire simple, où les fluctuations d'amplitude sont remplacées par des segments de droite dès que la dérivée du signal devient significative. Le résultat est un signal modélisé constitué d'une suite de segments de droite, qui sont ensuite très simples à traiter : par exemple, une onde monophasique est formée d'une suite de deux segments de sens opposés, et une onde biphasique de trois segments de sens opposés.

Cette dernière technique est très efficace, mais atteint ses limites lorsqu'elle s'applique à des cas particuliers comme par exemple des ondes de basse fréquence bruitées, qui conduisent à une surdécomposition en segments multiples, difficiles à analyser ensuite.

L'un des buts de l'invention est de proposer un dispositif d'analyse d'un signal, notamment d'un signal physiologique préalablement recueilli par un dispositif médical actif, qui puisse, notamment dans le cas d'un signal ECG, pallier les inconvénients des techniques jusqu'à présents utilisées.

Le but de l'invention est de proposer un tel dispositif qui, tout en faisant preuve d'une efficacité au moins égale à celle des meilleures techniques développées jusqu'à présent, puisse mettre en oeuvre avec des ressources informatiques restreintes (puissance du processeur et ressources mémoire), par exemple celles disponibles avec un micro-ordinateur de bureau ou portable traditionnel, de manière que le praticien puisse disposer des résultats de l'analyse automatique dans un laps de temps très court, typiquement quelques minutes au plus, après le transfert des données mémorisées par l'enregistreur Holter.

À cet effet, l'invention propose un dispositif d'analyse du type précité, c'est-à-dire un dispositif d'analyse d'un signal dont les variations définissent une fonction monodimensionnelle, ce signal ayant été préalablement filtré, échantillonné et numérisé. Un tel dispositif comprend des moyens de mémorisation du signal numérisé et d'analyse du signal mémorisé, avec des moyens extracteurs, aptes à décomposer le signal en une pluralité de N ondes élémentaires, et des moyens classificateurs, aptes à reconnaître au moins un paramètre caractéristique de chacune des N ondes élémentaires, et à attribuer un label standardisé, choisi parmi une pluralité de labels prédéterminés, en fonction du ou desdits paramètres caractéristiques ainsi reconnus.

Selon l'invention, les moyens extracteurs sont des moyens aptes à décomposer le signal en N fonctions bosse paramétrées, où chaque fonction bosse est une fonction continue définie sur trois intervalles successifs par, respectivement, une première fonction paramétrée monotone, une fonction affine, et une seconde fonction paramétrée monotone, lesdites fonctions paramétrées monotones étant l'une croissante, l'autre décroissante.. Selon diverses caractéristiques subsidiaires avantageuses :
- la dimension de ladite fonction monodimensionnelle est une dimension temporelle ;
- ledit signal est un signal électrocardiographique formant une onde de type PQRST ;
- ladite fonction affine est une fonction à pente nulle, et chacune desdites fonctions paramétrées est une demi-gaussienne, respectivement croissante et décroissante ; lesdits paramètres caractéristiques peuvent alors être les cinq paramètres constitués par les demi-écarts-type de chacune des deux demi-gaussiennes, la longueur de l'intervalle de définition de la partie affine, la position en ordonnée de cet intervalle, et l'amplitude au sommet des demi-gaussiennes ;
- les moyens extracteurs comprennent : des moyens de sélection, aptes à rechercher, parmi une pluralité de bosses-type réunies dans une bibliothèque de bosses-type prédéterminées, et pour chacune des N ondes élémentaires, la bosse-type respective la plus pertinente à l'égard du signal à décomposer, et des moyens d'adaptation des paramètres de chacune des N bosses-type ainsi déterminées par les moyens sélecteurs, aptes à minimiser l'écart entre le signal et la composition des N bosses-type paramétrées ;
- dans ce dernier cas, avantageusement, les moyens de sélection recherchent par orthogonalisation ladite bosse-type respective la plus pertinente et/ou les moyens d'adaptation adaptent lesdits paramètres par optimisation non-linéaire sous contraintes ;
- les moyens classificateurs opèrent par mise en oeuvre de chaînes de Markov cachées ;

Dans le cas particulier indiqué plus haut où le signal est un signal électrocardiographique formant une onde de type PQRST :
- les moyens d'analyse du signal mémorisé comprennent en outre des moyens soustracteurs, aptes à soustraire du signal mémorisé au moins l'une des N ondes élémentaires déterminées par les moyens extracteurs et portant un label donné, attribué par les moyens classificateurs ;
- lesdites ondes élémentaires sont au nombre de cinq, lesdits labels prédéterminés pouvant alors être ceux des ondes P, Q, R, S et T du signal électrocardiographique ;
- le signal électrocardiographique est un signal obtenu par analyse PCA et projection des composantes principales sur un axe significatif, notamment un axe d'amplitude maximale recalculé dynamiquement ;
- le dispositif comprend des moyens pour déterminer la variabilité au cours du temps d'au moins un facteur spécifique d'au moins l'une des N ondes élémentaires déterminées par les moyens extracteurs, notamment l'amplitude de l'onde T, l'amplitude de l'onde P ou la direction d'un axe significatif déterminé par analyse PCA ;
- le dispositif comprend des moyens pour déterminer une corrélation temporelle d'un facteur spécifique entre au moins deux des N ondes élémentaires déterminées par les moyens extracteurs, notamment l'intervalle temporel entre l'onde QRS et l'onde T ou l'intervalle PR.

On va maintenant décrire plus en détail un exemple de mise en oeuvre de l'invention, en référence aux figures annexées.

La figure 1, précitée, illustre un complexe PQRST d'un signal ECG.

La figure 2 illustre les éléments constitutifs d'une fonction bosse.

La figure 3 illustre les éléments constitutifs et les paramètres correspondants d'une fonction bosse particulière avantageusement utilisée pour la mise en oeuvre de l'invention.

Les figures 4a, 4b et 4c montrent, respectivement, le résultat de la décomposition réalisée selon l'invention, la composition des ondes donnant le signal modélisé, et le signal originel correspondant.

L'idée de départ de l'invention consiste à utiliser un algorithme rapide et fiable, pouvant être mis en oeuvre avec des ressources informatiques limitées, en opérant la modélisation du signal physiologique par décomposition en bosses, la "bosse" étant une notion mathématique déjà connue en elle-même mais qui n'avait, jusqu'à présent, jamais été proposée dans le cadre de l'analyse d'un signal physiologique.

Une bosse est, comme illustré figure 2, une fonction monotone, paramétrée, définie sur trois intervalles successifs par une première fonction paramétrée croissante G1, une fonction affine D et une seconde fonction paramétrée décroissante G2.

Selon l'invention, le dispositif d'analyse cherche à composer un ensemble de bosses tel que le graphe de la somme de ces bosses soit aussi semblable que possible à un signal monodimensionnel connu.

L'optimisation des paramètres de chaque bosse est opérée par toute méthode mathématique appropriée permettant d'obtenir un graphe aussi proche que possible du signal originel à modéliser.

Sur la figure 4, on a ainsi illustré, dans le cas particulier d'un battement d'un signal ECG :
1°) l'ensemble des bosses, ici au nombre de cinq, élaboré de la manière que l'on décrira plus bas,
2°) le graphe résultant de la composition de ces cinq bosses, et
3°) le signal originel qui, comme on peut le voir, est très proche de la modélisation obtenue en (b).

Avantageusement, on utilise un type de bosse particulier, illustré figure 3, dérivé de la définition générale donnée plus haut en référence à la figure 2.

Ce type particulier de bosse est défini par un nombre de paramètres réduit (cinq en l'espèce) et, en pratique, la modélisation obtenue s'avère extrêmement fiable et proche du signal originel dans le cas de l'analyse d'un signal ECG, malgré le nombre réduit des paramètres de définition de la bosse.

À cet effet, on utilise comme partie affine D de la bosse un segment horizontal (c'est-à-dire de pente nulle) et, afin de pouvoir exécuter l'étape d'adaptation des paramètres par des algorithmes classiques d'optimisation multidimensionnelle, les fonctions monotones G1 et G2 sont ici des demi-gaussiennes d'amplitude égale A.

Sous ces conditions, la bosse est une fonction continue, définie par cinq paramètres et dérivable par rapport à chacun de ces paramètres, qui sont :
- µ : position temporelle, par exemple, la position en ordonnée du milieu du segment D ;
- σ1 : demi-écart-type de la première gaussienne G1 ;
- σL : longueur du segment D ;
- σ2 : demi-écart-type de la seconde gaussienne G2 ; et
- A : amplitude de la fonction.

La décomposition du signal temporel en bosses est opérée de manière itérative en deux étapes :
1°) Sélection de la bosse la plus pertinente par un algorithme de sélection appliqué à un ensemble de bosses préalablement construites et conservées dans une bibliothèque de bosses-types dans la mémoire de l'ordinateur ; cette étape de sélection peut avantageusement utiliser une méthode d'orthogonalisation en elle-même connue (voir par exemple M J Korenberg, S A Billings, Y P Liu et coll., Orthogonal parameter estimation for non-linear stochastic systems, International Journal of Control, 48, 193-210, 1988).
2°) Adaptation des paramètres de la bosse qui a été sélectionnée à l'étape précédente, c'est-à-dire recherche des cinq paramètres µ, σ1, σL, σ2, et A indiqués plus haut, de manière que la bosse particulière finalement obtenue se rapproche le plus possible du signal originel à modéliser ; cette étape d'adaptation peut avantageusement utiliser une méthode d'optimisation non linéaire sous contraintes, en elle-même connue (voir par exemple M Minoux, Programmation Mathématique (1983), Dunod, 1983)

Ainsi, la modélisation en N bosses d'un signal se fera en N fois deux étapes. Le nombre N maximum de bosses est soit défini à l'avance, soit adapté dynamiquement en fonction de la précision de la modélisation recherchée.

Dans le cas courant d'un signal ECG, un battement modélisé sur cinq bosses s'avère en pratique satisfaisant : la limite de cinq bosses correspond au cas simple où une bosse représente une onde caractéristique P, Q, R, S ou T de l'enregistrement ECG, comme illustré sur la figure 4a (la figure 4b représentant l'onde modélisée obtenue par composition des cinq bosses de la figure 4a et la figure 4c le signal originel analysé par le dispositif de l'invention).

Le signal modélisé en bosses est ensuite analysé pour y repérer les ondes caractéristiques de l'activité cardiaque.

Chaque bosse se voit alors attribuer une étiquette (P,Q,R, S, T ou autre) suivant sa forme et son emplacement par rapport aux autres bosses. On peut avantageusement utiliser pour cette labellisation une méthode à base de chaînes de Markov cachées (CMC ou HMM, *Hidden Markov Models*), méthode en elle-même connue et décrite par exemple par L R Rabiner, A Tutorial on Hidden Markov Models and Selected Applications in Speech Recognition, Proceedings of the IEEE, 77 (2), 257-286 (1989).

Cet étiquetage des bosses permet de reconnaître les différentes composantes d'un complexe PQRST typique et ainsi de détecter aisément les ondes atypiques, qui sont celles qui, précisément, présentent le plus d'intérêt pour le diagnostic des troubles du rythme.

Chaque onde de l'ECG ayant été identifiée, il est possible de mesurer chaque paramètre caractéristique d'une onde donnée et - surtout - de l'étudier de manière dynamique. Les courbes obtenues peuvent être analysées une à une ou en relation avec d'autres, car la modélisation procure des formes stabilisées qui permettent des corrélations efficaces.

On peut ainsi analyser de façon pertinente la variabilité de l'onde T par mesure de ses variations d'amplitude et/ou de son décalage temporel par rapport à l'onde QRS. On peut également analyser les paramètres de la bosse représentant l'onde T, évaluer la variabilité de l'intervalle PR, de l'amplitude de l'onde P, etc.

La reconnaissance des ondes permet également de soustraire une onde connue à un signal ; cette opération est très utile pour démasquer une onde de faible amplitude (par exemple, l'onde P) qui survient de façon synchrone à une onde de forte amplitude (Q,R,S ou T).

Cette soustraction automatique d'onde sur le signal permet de faire apparaître des signaux sous-jacents, par exemple des ondes P très précoces, survenant juste après le QRS précédent et qui restaient très souvent masquées avec les techniques d'analyse jusqu'à présent utilisées.

La labellisation des ondes P fournit des résultats qui permettront ensuite au médecin d'améliorer considérablement le diagnostic.

L'exemple de mise en oeuvre de l'invention que l'on vient de décrire peut se prêter à de nombreuses variantes.

Ainsi, plutôt que de modéliser directement le signal recueilli sur chacune des voies ECG après filtrage, il est avantageux d'effectuer au préalable, de façon dynamique (c'est-à-dire pour chaque battement), une analyse en composantes principales (ACP ou PCA, *Principal Component Analysis*).

Cette technique, en elle-même connue (voir par exemple I T Jolliffe, Principal Component Analysis, Springer, 1986) consiste à utiliser les signaux simultanément obtenus sur les voies X, Y et Z et enregistrés sur plusieurs pistes, en recherchant dans un espace tridimensionnel un axe significatif d'amplitude maximale (axe PCA1) et en exprimant la variation temporelle du battement par sa projection sur cet axe principal, dont la position est recalculée à chaque battement.

Ce prétraitement permet en particulier de modéliser l'ECG sur une piste seulement, contenant le maximum d'informations, plutôt que sur l'ensemble des pistes originales.

L'analyse en composantes principales du complexe PQRST (ou du complexe QRS et/ou de l'onde P) permet en outre d'obtenir en permanence le plan de projection des signaux électriques cardiaques, plan qui est lié principalement à la position du coeur dans l'espace. Une analyse du mouvement de ce plan ou, plus simplement, de l'axe de la composante principale (axe PCA1), permet d'obtenir une image du mouvement du coeur qui peut être utilisée à différentes fins telles que :
- la détection de la respiration (qui a la propriété de déplacer le coeur à chaque cycle) et la discrimination entre respiration thoracique ou abdominale ;
   En effet, le déplacement du coeur, intervenant dans des axes ou des courbes différents, se traduit par une modulation d'un ou plusieurs angles des composantes principales qui, analysés à l'aide d'outils mathématiques appropriés, permet l'extraction d'une ou plusieurs courbes de respiration, abdominales et thoraciques par exemple ;
- la détection de la position du corps, la pesanteur provoquant des changements anatomiques qui sont transmis au coeur et au signal ECG ;
- la compensation des déformations du signal ECG introduites par les changements de position qui déplacent le coeur : connaissant la position électrique, un calcul matriciel dynamique appliqué aux dérivations XYZ ou aux dérivations standard permet de compenser l'influence de ces changements et de recréer un signal stabilisé. Cette stabilité est extrêmement utile pour diverses analyses, en particulier les comparaisons entre deux ECG enregistrés ;
- la création d'un ECG original par projection du signal spatial selon les trois composantes X, Y et Z. Par rapport à un ECG XYZ, cet ECG original présente deux avantages, à savoir :(i) une très grande stabilité, puisqu'il n'est pas soumis aux variations de position du coeur et (ii) un niveau du signal qui est maximum dans la première voie correspondant à la projection sur l'axe principal. Il peut être complété par une information sur les angles des axes de projection, qui représentent alors les changements positionnels du coeur, c'est-à-dire l'effet des changements de position corporels et de la respiration.

Ces différentes techniques résultant de l'analyse PCA sont en elles-mêmes connues, mais la mise en oeuvre de l'invention permet d'en améliorer grandement l'efficacité, grâce notamment à l'étiquetage des bosses. L'étiquetage des bosses permet d'améliorer l'efficacité de cette analyse de type PCA en la réalisant si besoin est sur une onde particulière.

L'invention est notamment applicable à des enregistreurs ambulatoires externes tels que les modèles SYNEFLASH et SYNEVIEW de la société ELA Médical, Montrouge, France.

Elle est également applicable à des appareils implantés tels que les dispositifs implantés à microprocesseur, également commercialisés par ELA Médical, Montrouge, France, auxquels il est possible de transmettre par télémétrie des logiciels qui seront conservés en mémoire et exécutés pour mettre en oeuvre les fonctions de l'invention décrites ci-dessus.

L'adaptation de ces appareils à la mise en oeuvre de l'invention est à la portée du spécialiste de ces techniques et ne sera pas décrite en détail.

## Revendications

1. Un dispositif d'analyse d'un signal physiologique dont les variations définissent une fonction monodimensionnelle, ce signal ayant été préalablement recueilli, filtré, échantillonné et numérisé,
ce dispositif comprenant :
- des moyens de mémorisation du signal numérisé, et
- des moyens d'analyse du signal mémorisé, comprenant :
· des moyens extracteurs, aptes à décomposer le signal en une pluralité de N ondes élémentaires, et
· des moyens classificateurs, aptes à reconnaître au moins un paramètre caractéristique de chacune des N ondes élémentaires, et à attribuer un label standardisé, choisi parmi une pluralité de labels prédéterminés, en fonction du ou desdits paramètres caractéristiques ainsi reconnus,
ce dispositif étant **caractérisé en ce que** les moyens extracteurs sont des moyens aptes à décomposer le signal en N fonctions bosse paramétrées (1-5), où chaque fonction bosse est une fonction continue définie sur trois intervalles successifs par, respectivement, une première fonction paramétrée monotone (G1), une fonction affine (D), et une seconde fonction paramétrée monotone (G2), lesdites fonctions paramétrées monotones étant l'une croissante, l'autre décroissante.

2. Le dispositif de la revendication 1 dans lequel ledit signal physiologique est un signal préalablement recueilli par un dispositif médical actif.

3. Le dispositif de la revendication 1 dans lequel la dimension de ladite fonction monodimensionnelle est une dimension temporelle.

4. Le dispositif de la revendication 3 dans lequel ledit signal est un signal électrocardiographique formant une onde de type PQRST.

5. Le dispositif de la revendication 1 dans lequel ladite fonction affine (D) est une fonction à pente nulle.

6. Le dispositif de la revendication 1 dans lequel chacune desdites fonctions paramétrées (G1, G2) est une demi-gaussienne, respectivement croissante et décroissante.

7. Le dispositif des revendication 5 et 6 prises en combinaison, dans lequel lesdits paramètres caractéristiques sont les cinq paramètres constitués par les demi-écarts-type (σ1, σ2) de chacune des deux demi-gaussiennes, la longueur (σL) de l'intervalle de définition de la partie affine, la position en ordonnée (µ) de cet intervalle, et l'amplitude au sommet (A) des demi-gaussiennes.

8. Le dispositif de la revendication 1 dans lequel les moyens extracteurs comprennent :
- des moyens de sélection, aptes à rechercher, parmi une pluralité de bosses-type réunies dans une bibliothèque de bosses-type prédéterminées, et pour chacune des N ondes élémentaires, la bosse-type respective la plus pertinente à l'égard du signal à décomposer, et
- des moyens d'adaptation des paramètres de chacune des N bosses-type ainsi déterminées par les moyens sélecteurs, aptes à minimiser l'écart entre le signal et la composition des N bosses-type paramétrées.

9. Le dispositif de la revendication 8 dans lequel les moyens de sélection recherchent par orthogonalisation ladite bosse-type respective la plus pertinente.

10. Le dispositif de la revendication 8 dans lequel les moyens d'adaptation adaptent lesdits paramètres par optimisation non-linéaire sous contraintes.

11. Le dispositif de la revendication 1 dans lequel les moyens classificateurs opèrent par mise en oeuvre de chaînes de Markov cachées.

12. Le dispositif de la revendication 4 dans lequel les moyens d'analyse du signal mémorisé comprennent en outre des moyens soustracteurs, aptes à soustraire du signal mémorisé au moins l'une des N ondes élémentaires déterminées par les moyens extracteurs et portant un label donné, attribué par les moyens classificateurs.

13. Le dispositif de la revendication 4 dans lequel lesdites ondes élémentaires sont au nombre de cinq.

14. Le dispositif de la revendication 13 dans lequel lesdits labels prédéterminés sont ceux des ondes P, Q, R, S et T du signal électrocardiographique.

15. Le dispositif de la revendication 4 dans lequel le signal électrocardiographique est un signal obtenu par analyse PCA et projection des composantes principales sur un axe significatif.

16. Le dispositif de la revendication 15 dans lequel ledit axe significatif est un axe d'amplitude maximale recalculé dynamiquement.

17. Le dispositif de la revendication 14 dans lequel le dispositif comprend en outre des moyens pour déterminer la variabilité au cours du temps d'au moins un facteur spécifique d'au moins l'une des N ondes élémentaires déterminées par les moyens extracteurs.

18. Le dispositif de la revendication 14 dans lequel le dispositif comprend en outre des moyens pour déterminer une corrélation temporelle d'un facteur spécifique entre au moins deux des N ondes élémentaires déterminées par les moyens extracteurs.

19. Le dispositif de la revendication 17 ou 18 dans lequel ledit facteur spécifique est un facteur du groupe formé par : l'amplitude de l'onde T ; l'intervalle temporel entre l'onde QRS et l'onde T ; l'intervalle PR ; l'amplitude de l'onde P ; et la direction d'un axe significatif déterminé par analyse PCA.

## Claims

1. A device for analyzing a physiological signal, the variations of which define a one dimensional function, this signal having been previously collected, filtered, sampled and digitized,
this device comprising:
- means for memorizing the digitized signal, and
- means for analyzing the memorized signal, comprising:
extracting means able to decompose the signal into a plurality of N elementary waves, and
classifying means able to recognize at least one characteristic parameter of each of the N elementary waves, and to allot a standardized label selected from among a plurality of predetermined labels according to the one or more such recognized characteristic parameters,
this device being **characterised in that** the extracting means are means able to decompose the signal into N parameterized bump functions (1-5), wherein each bump function is a continuous function defined over three successive intervals by, respectively, a first monotonic parameterized function (G1), an affine function (D), and a second monotonic parameterized function (G2), said monotonic parameterized functions being one increasing and the other one decreasing.

2. The device of claim 1, wherein said physiological signal is a signal previously collected by an active medical device.

3. The device of claim 1, wherein the dimension of said one dimensional function is a temporal dimension.

4. The device of claim 3, wherein said signal is an electrocardiographic signal forming a wave of the PQRST type.

5. The device of claim 1, wherein said affine function (D) is a function with no slope.

6. The device of claim 1, wherein each of said parameterized functions (G1, G2) is a respectively increasing and decreasing half-Gaussian function.

7. The device of claims 5 and 6 in combination, wherein said characteristic parameters are the five parameters consisting of half the standard deviations (σ1, σ2) of each of the two half-Gaussian functions, the length (σL) of the definition interval of the affine part, the ordinate position (µ) of this interval, and the peak amplitude (A) of the half-Gaussian functions.

8. The device of claim 1, wherein the extracting means comprise:
- selecting means, able to search, amongst a plurality of bump types gathered in a library of predetermined bump types, and for each of the N elementary waves, for the respective bump type that is the most relevant in regard to the signal to be decomposed, and
- means for adapting the parameters of each of the N bump-types such determined by the selecting means, able to minimise the deviation between the signal and the composition of the N parameterized bump-types.

9. The device of claim 8, wherein the selecting means search for said respective most relevant bump-type by orthogonalisation.

10. The device of claim 8, wherein the adapting means adapt said parameters by a non-linear optimization under constraints.

11. The device of claim 1, wherein the classifying means operate by implementing hidden Markov chains.

12. The device of claim 4, wherein the means for analysing the memorised signal furthermore comprise subtracting means able to subtract, from the memorised signal, at least one of the N elementary waves determined by the extracting means and carrying a given label allotted by the classifying means.

13. The device of claim 4, wherein said elementary waves are five waves.

14. The device of claim 13, wherein said predetermined labels are those of the P, Q, R, S and T waves of the electrocardiographic signal.

15. The device of claim 4, wherein the electrocardiographic signal is a signal obtained by a PCA analysis and projection of the principal components onto a significant axis.

16. The device of claim 15, wherein said significant axis is a dynamically recalculated axis of maximum amplitude.

17. The device of claim 14, wherein the device further comprises means for determining the variability over time of at least one specific factor of at least one of the N elementary waves determined by the extracting means.

18. The device of claim 14, wherein the device further comprises means for determining a temporal correlation of a specific factor between at least two of the N elementary waves determined by the extracting means.

19. The device of claim 17 or 18, wherein said specific factor is a factor of the group formed by: the amplitude of the T wave; the temporal interval between the QRS complex and the T wave; the PR interval; the amplitude of the P wave; and the direction of a significant axis determined by PCA analysis.

## Patentansprüche

1. Vorrichtung zur Analyse eines physiologischen Signals, dessen Variationen eine eindimensionale Funktion definieren, wobei dieses Signal vorab aufgenommen, gefiltert, abgetastet und digitalisiert wurde,
wobei diese Vorrichtung folgendes umfasst:
- Mittel zur Speicherung des digitalisierten Signals, und
- Mittel zur Analyse des gespeicherten Signals, mit:
· Extraktionsmitteln, die geeignet sind, das Signal in eine Mehrzahl von N Elementarwellen zu zerlegen, und
· Klassifikationsmitteln, die geeignet sind, wenigstens einen charakteristischen Parameter jeder der N Elementarwellen zu erkennen, und ein standardisiertes Label zuzuteilen, das aus einer Mehrzahl von vorbestimmten Labeln ausgewählt ist, in Abhängigkeit des oder der so erkannten charakteristischen Parameter,
wobei diese Vorrichtung **dadurch gekennzeichnet ist, dass** die Extraktionsmittel Mittel sind, die geeignet sind, das Signal in N parametrisierte Buckelfunktionen (1-5) zu zerlegen, wo jede Buckelfunktion eine stetige Funktion ist, die über drei aufeinander folgende Intervalle jeweils durch eine erste parametrisierte monotone Funktion (G1), eine affine Funktion (D) und eine zweite parametrisierte monotone Funktion (G2) definiert ist, wobei bei den monotonen parametrisierten Funktionen die eine steigend und die andere fallend ist.

2. Vorrichtung nach Anspruch 1, bei welcher das physiologische Signal ein Signal ist, das vorab durch eine aktive medizinische Vorrichtung aufgenommen wurde.

3. Vorrichtung nach Anspruch 1, bei welcher die Dimension der eindimensionalen Funktion eine Zeitdimension ist.

4. Vorrichtung nach Anspruch 3, bei welcher das Signal ein elektrokardiographisches Signal ist, das einen Komplex vom PQRST-Typ bildet.

5. Vorrichtung nach Anspruch 1, bei welcher die affine Funktion (D) eine Funktion mit einer Steigung von null ist.

6. Vorrichtung nach Anspruch 1, bei welcher jede der parametrisierten Funktionen (G1, G2) eine jeweils steigende und fallende halbe Gauß-Funktion ist.

7. Vorrichtung der Ansprüche 5 und 6 in Kombination, bei welcher die charakteristischen Parameter die fünf Parameter sind, die aus den halben Standardabweichungen (σ1, σ2) einer jeden der zwei halben Gauß-Funktionen, der Länge (σL) des Definitionsintervalls des affinen Abschnitts, der Ordinatenposition (µ) diese Intervalls und der Amplitude am Höhepunkt (A) der halben Gauß-Funktionen bestehen.

8. Vorrichtung nach Anspruch 1, bei welcher die Extraktionsmittel folgendes umfassen:
- Selektionsmittel, die geeignet sind, innerhalb einer Mehrzahl an typischen Buckelfunktionen, die in einer Bibliothek von vorbestimmten typischen Buckelfunktionen gesammelt sind, und für jede der N Elementarwellen die im Hinblick auf das zu zerlegende Signal zutreffendste jeweilige typische Buckelfunktion zu suchen, und
- Mittel zur Anpassung der Parameter einer jeden der so durch die Selektionsmittel bestimmten N typischen Buckelfunktionen, die geeignet sind, die Abweichung zwischen dem Signal und der Zusammensetzung der N parametrisierten typischen Buckelfunktionen zu minimieren.

9. Vorrichtung nach Anspruch 8, bei welcher die Selektionsmittel durch Orthogonalisierung die jeweilige zutreffendste typische Buckelfunktion suchen.

10. Vorrichtung nach Anspruch 8, bei welcher die Anpassungsmittel die Parameter durch nicht lineare Optimierung unter Bedingungen anpassen.

11. Vorrichtung nach Anspruch 1, bei welcher die Klassifikationsmittel durch Einsatz von versteckten Markow-Ketten arbeiten.

12. Vorrichtung nach Anspruch 4, bei welcher die Mittel zur Analyse des gespeicherten Signals außerdem Subtraktionsmittel umfassen, die geeignet sind, vom gespeicherten Signal wenigstens eine der N Elementarwellen abzuziehen, die durch die Extraktionsmittel bestimmt wurden und mit einem gegebenen Label versehen sind, das durch die Klassifikationsmittel zugeteilt wurde.

13. Vorrichtung nach Anspruch 4, bei welcher die Elementarwellen fünf an der Zahl sind.

14. Vorrichtung nach Anspruch 13, bei welcher die vorbestimmten Label diejenigen der P-, Q-, R-, S- und T-Wellen des elektrokardiographischen Signals sind.

15. Vorrichtung nach Anspruch 4, bei welcher das elektrokardiographische Signal ein Signal ist, das durch eine Hauptkomponentenanalyse und Projektion der Hauptkomponenten auf eine signifikative Achse erhalten wird.

16. Vorrichtung nach Anspruch 15, bei welcher die signifikative Achse eine dynamisch neu berechnete Achse maximaler Amplitude ist.

17. Vorrichtung nach Anspruch 14, bei welcher die Vorrichtung außerdem Mittel zur Bestimmung der zeitlichen Veränderlichkeit wenigstens eines spezifischen Faktors wenigstens einer der durch die Extraktionsmittel bestimmten N Elementarwellen umfasst.

18. Vorrichtung nach Anspruch 14, bei welcher die Vorrichtung außerdem Mittel zur Bestimmung einer zeitlichen Korrelation eines spezifischen Faktors zwischen wenigstens zwei der durch die Extraktionsmittel bestimmten N Elementarwellen umfasst.

19. Vorrichtung nach Anspruch 17 oder 18, bei welcher der spezifische Faktor ein Faktor aus der Gruppe ist, die durch folgendes gebildet wird: die Amplitude der T-Welle; das Zeitintervall zwischen dem QRS-Komplex und der T-Welle; das PR-Intervall; die Amplitude der P-Welle; und die Richtung einer durch Hauptkomponentenanalyse bestimmten signifikativen Achse.
